(19) Europäisches Patentamt

European Patent Office

Office européen des brevets

(11) **EP 2 607 472 A1**

(12) **EUROPEAN PATENT APPLICATION**
published in accordance with Art. 153(4) EPC

(43) Date of publication:
   **26.06.2013 Bulletin 2013/26**

(51) Int Cl.:
   **C12M 1/00** (2006.01)          **C12P 7/40** (2006.01)

(21) Application number: **11817591.8**

(22) Date of filing: **17.08.2011**

(86) International application number:
   **PCT/BR2011/000287**

(87) International publication number:
   **WO 2012/021955 (23.02.2012 Gazette 2012/08)**

(84) Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**

(30) Priority: **19.08.2010 BR PI1004963**

(71) Applicant: **Companhia Refinadora da Amazônia
   66825-000 Belém - PA (BR)**

(72) Inventor: **YOSHIDA, Paulo
   22630-010 Rio De Janeiro - RJ (BR)**

(74) Representative: **Ström & Gulliksson AB
   P.O. Box 4188
   203 13 Malmö (SE)**

(54) **STSTEM AND PROCESS FOR CORRECTING CONSTANT VOLUME ACIDITY OF FERMENTATIVE MEDIA FOR PRODUCING ORGANIC ACIDS**

(57)    A system (100, 200) is described for correcting constant volume acidity of fermentative media for the production of organic acids, which comprises a fermenter (1) provided with a pH sensor (P1), a filtration module (2) provided with a filtering element, an addition vessel (3) provided with a main metering element (4), and a heat exchanger (5). Operation of the system (100, 200) is controlled by a controller (CT). As soon as the sensor (P1) detects a reduction in pH to below the ideal values for producing organic acid, the controller (CT) calculates the amount of fermentative medium to be withdrawn from the fermenter (1) and said amount is conveyed to the vessel (3) in order to be alkalinized and returned to the system. Arrangements of the system with more than one fermenter are also described. The process for correcting acidity used by the system (100, 200) of the invention is likewise described.

FIGURE 1

EP 2 607 472 A1

**Description**

FIELD OF THE INVENTION

The present invention belongs to the field of systems and processes for obtaining organic acids by fermentation and more specifically, to a system and process for correcting constant volume acidity of fermentative media.

BACKGROUND OF THE INVENTION

It is well known in scientific and technical literature (Benninga, H. history of making lactic acid: a chapter in the history of biotechnology. Kluwer Academic Press, The Nederlands, 1989) that anaerobic fermentation of sugars such as glucose, fructose and sucrose, has lactic acid as one of its final products. Other types of organic acids such as citric acid, maleic acid, fumaric acid, adipic acid, succinic acid, tartaric acid, and others, may also be generated in similar processes. *Lactobacillus sp.* bacteria constitute one of the most used microorganisms in the preparation of organic acids by fermentation, providing an alternative to the methods of synthesis using products derived from oil. In case of lactic acid fermentation, the process can only take place in well-defined process ranges. For example, it is observed that any significant variation in pH values of the fermentative medium results in low productivity or even in full interruption of the production. Furthermore, according to the patent document US 5,766,439, in the name of Aharon and Willian, the fermentative process for producing such acids *per* se involves a modification in the medium acidity and also in carbon dioxide release from bacterial activity. Consequently, the process can be self-extinguished if there is no means of adjusting fermentation's pH to suitable values.

Several approaches to solve this problem have been reported. For example, in document US 2004/0033573, Norddahl and col. claim a method for producing lactic acid in liquid sugar-rich fermentative medium by using acid-producing bacteria, which results in lactate salts, typically sodium, ammonium or potassium lactate. During the fermentative process, the pH of the fermentative medium is kept between 5 and 7 by adding appropriate amount of base. Bases which may be added are ammonia, typically ammonia gas, NaOH and KOH, or a mixture thereof. All these bases form soluble salts with lactic acid. At this point, applicants argue that using ammonia as a base has the advantage of acting as a nitrogen source for the microorganisms as well as being cheaper than other types of base. On the other hand, NaOH or KOH bases are more easily recovered in a subsequent purification step. This process has the clear disadvantage of ammonia volatility, which can permeate through the membranes used for separating lactic acid and, moreover, every addition of a certain volume of basic solution to the fermenter implies the need of removing the same volume in a subsequent operation, which leads to a consequent cost increase in the process as a whole.

Patent document US 4,882,277 claims a process wherein glucose is continuously fermented to lactate and the resulting lactic acid is subsequently extracted from the solution by electrodialysis, where the pH within the fermenter is controlled by removing lactic acid at the same rate in which is formed as the fermenter content is recirculated in the electrodialysis unit. The disadvantage of this process relates to the fact that the bacteria present in the fermenter are absorbed in the ion exchange membranes present in the electrodialysis unit, which causes increased electrical resistance in the unit and consequently leads to increased power consumption in the process. Additionally, cell death should be taken into consideration so that process total productivity should be affected by reduction in the amount of producing cells. Patent document US 2006/0094093 claims the process for producing lactic acid at low pH values by using a homolactic bacterium derived from acid-tolerant corn, which is able to produce high levels of free lactic acid. The acid-tolerant bacteria are able to produce at least about 25 g/L of free lactic acid. Such type of bacteria generally can also produce about 50 g/L of lactate in the fermentative medium in an average incubation pH of no more than 4.2. The process productivity described is such that the process is normally conducted so as to produce about 1.0 to 3.0 $g.L^{-1}.h^{-1}$ in a medium whose average incubation pH is less than 4.0. Even using bacteria which are able to produce lactic acid at pH values lower than usual, the process in reference does not eliminate the need of maintaining pH range by the addition of bases or buffering agents. In this case, applicants suggest that lactate in salt form can return to the fermentative vessel and act as a pH buffer in the solution and prevent pH decrease in the reaction medium below the desired values. However, in this process, lactate salt reflux does not discard the addition of other neutralizing agents such as calcium carbonate, sodium hydroxide and/or sodium bicarbonate which, besides increasing the cost, can significantly decrease process total productivity. Furthermore, the cost of the bacteria is greater in relation to *Lactobacillus sp.,* normally used for fermentative production of lactic acid.

Thus, it can be noted that in all the above-described situations, the possibility of high concentrations of certain salts, e.g. sodium cations, may cause inhibition of the fermentative process and the type or amount of salts present may impair the efficiency of fermentation.

In *Neutralization/recovery of lactic acid from Lactococcus lactis: effects on biomass, lactic acid, and nisin production* (World Journal of Microbiology & Biotechnology, Vol. 13, 1997) Van't Hul and Gibbons have described the process for obtaining lactic acid and other metabolic products by using an automated pH control with the addition of ammonium hydroxide. According to the authors, the use of the described procedure with 6 mol/L of NH$_4$OH solution to neutralize the reaction medium led to significant increase in cell production of nisin and lactic acid, but does take into consideration the volume increase caused by the addition of the neutralizing agent which, at the end of the process, will result in a higher energy consumption.

Thus, it would be interesting if the art could provide a process for correcting constant volume acidity in fermentative media based on a system of withdrawal of a certain volume of medium, filtration of such volume, alkalinization and subsequent addition of such volume back to the reaction medium so as to control fermentative medium pH within the required conditions for the operation and production of said acids.

SUMMARY OF THE INVENTION

In a broad way, the present invention relates to a system for correcting constant volume acidity in fermentative media consisting of at least one fermenter (1) having at least one sensor for determining real time fermentative medium pH (P1), a filtration module (2), a vessel for the addition of a base (3), a first pump for pumping fermentative broth from the fermenter to said filtration module (B1), a second pump for pumping the basified fermentative broth fraction back to the fermenter (B2) and a heat exchanger (5), the volumetric ratios of alkaline solution for pH correction to be added to the fraction withdrawn from the fermenter being established by a controller connected to said at least one sensor (P1), said first pump (B1), said second pump (B2), said heat exchanger (5) and base dosimeter as defined herein (4) in order to allow that the fermenter (1) outflow rate, the amount of base added to the solution in said addition vessel (3) and the alkaline solution backflow rate to said fermenter (1) are appropriate and in accordance with the adjusting requirement of the medium pH at a given point in the organic acid production process.

The process for correcting constant volume acidity in fermentative media carried out with the aid of the present system is based on a procedure of withdrawal of certain medium volume, filtration of such volume, alkalinization and subsequent addition of such volume back to the reaction medium in order to control the pH of the fermentative medium under the required conditions for operation and production of said acids.

Thus, the present invention provides a system for correcting constant volume acidity in fermentative media, the system comprising at least one fermenter having at least one sensor, a vessel for the addition of a base and a base dosimeter, a first pump for pumping fermentative broth from the fermenter to a filtration module, a second pump for pumping the basified fermentative broth fraction back to the fermenter and a heat exchanger, the volumetric ratios of alkaline solution for pH correction to be added to the fraction withdrawn from the fermenter being established by a controller connected to said at least one sensor, said first pump, said second pump, said heat exchanger and the base dosing element.

The invention further provides a process for correcting constant volume acidity in fermentative media, said process being optimized by a reflux system of the culture medium under appropriate pH conditions, so that when the culture medium returns to the fermenter an acidity adjustment of the reaction medium with higher productivity occurs, without causing an increase in reaction volume.

An advantage of said process for correcting constant volume acidity in fermentative media of the present invention lies in that it provides a significant energy saving in the process as a whole.

Additionally, the process of the present invention provides a reduced environmental impact by saving inputs and energy.

BRIEF DESCRIPTION OF THE DRAWINGS

Figure 1 attached herein is a simplified flowchart of the process for correcting constant volume acidity in fermentative media of the invention.
Figure 2 attached herein is a simplified flowchart of the control layout of the process of the invention.
Figure 3 attached herein is a simplified flowchart of the process of the invention which is adapted to a set of reactors, so that the alkalinization process of the permeate can operate continuously.

DETAILED DESCRIPTION OF THE INVENTION

The present invention is based on the fact that adding alkaline substances to fermentative media for producing organic acids causes a significant increase in the fermentative medium volume, which necessarily involves an increase in the energy amount required to separate the synthesized lactic acid from the reaction medium thereof. According to the invention, part of the fermentative medium can be removed from the fermentative vessel, filtered for the retention of microorganisms, added with alkaline element and then returned to the fermentative medium in a volume which is defined so as to adjust the culture medium pH to values within the optimal operating limits.

Most microorganisms have an optimal range of pH values at which their metabolism is optimized, and thus the pH of a particular fermentation medium is a variable that strongly affects process productivity.

Microorganisms such as *Lactobacillus sp.* are conventionally employed as lactic acid producers, and in a fermentative process driven by these microorganisms, the pH values rapidly decrease with lactic acid production at levels which may inhibit cellular metabolism or cause cell death. Thus, it is well established in literature that the addition of alkaline agents to the fermentative medium is able to maintain the pH values at appropriate levels so that lactic acid production is maximal. Reaction medium pH values which are recommended for obtaining good lactic acid productivity are between 5.0 and 7.0 (see U.S patent document no. 5,510,526).

In addition, studies were undertaken in order to obtain organisms which are able to maintain high productivity of lactic acid at pH values between 3.0 and 4.8 (International Publication no. WO 99/19290; patent document US 2006/0094093).

When applied to weak acids such as lactic acid, other than those obtained in fermentative processes, the chemical equilibrium theory defines that, in solution, lactic acid dissociates according to the following equation (equation 1):

$$CH_3CH(OH)CO_2H \rightleftharpoons H^+ + CH_3CH(OH)CO_2^-$$

(equation 1)

According to this chemical equilibrium equation, the lactic acid production by microorganisms will increase the amount of dissociated acid, and consequently will cause a reduction in the culture medium pH, since the pH of a solution is related to the concentration of acids present in the medium according to equation 2 below:

$$pH = pKa + \log\left(\frac{[R(OH)COO^-]}{[R(OH)COOH]}\right)$$

(equation 2)

wherein pKa is the logarithm of the weak acid dissociation constant, while [R(OH)COO⁻] and [R(OH)COOH] are respectively the molar concentrations of the counterion and organic acid.

Thus, the addition of bases such as KOH, NaOH, $Ca(OH)_2$, $NH_4OH$, without being limited thereto, will cause an increase in the consumption of $H^+$ ions due to the dissociation equation represented by equation 3 below, and it has the solution pH increasing as a consequence.

$$M(OH)_x \rightleftharpoons M^{x+} + x(OH)^-$$

(equation 3)

Thus, if the added amount of base is equal to the produced amount of acid, the solution pH will be kept at a constant value which can be adjusted, preferably between 4.0 and 7.0, in order to maximize the production of organic acid by microorganisms.

Several fermentative media compositions for the culture of various types of microorganisms are proposed in the technical and scientific literature and they are already commercially available. One of the most commonly used available media is known as MRS (from Man, *Rogosa and Sharpe,* which are the inventors thereof), which has the following composition (mass/volume) in its more general version:

- 1.0% of peptone;
- 0.8% of animal tissue extract (usually bovine meat);
- 0.4% of yeast extract;
- 2.0% of glucose;

- 0.5% of sodium acetate;
- 0.1% of Polysorbate 80 (also known as Tween 80);
- 0.2% of potassium hydrogen sulfate;
- 0.2% of ammonium citrate;
- 0.02% of magnesium sulfate;
- 0.005% of manganese sulfate;
- pH adjusted to 6.2 at 24 $\pm$ 1 °C.

In this regard, see the reference below:

(http://www.bd.com/ds/technicalCenter/inserts/Lactoba cilli MRS Agar & Broth.pdf, in 11/19/2009).

Such composition is recommended for microorganism cultures such as: *Lactobacillus acidophilus, Lactobacillus casei, Lactobacillus fermentum, Escherichia* coli, *Pseudomonas aeruginosa,* among others.

Modifications and adaptations of the MRS medium are also now available on the market, in order to cover the types of microorganisms to be cultured.

The typical fermentation process occurs in a suitable temperature controlled fermenter in which microorganisms are kept by adjusting the broth composition under optimal conditions for the production of organic acid during periods of 18 to 36 hours.

The process temperature is kept between 25°C and 42°C. The fermenter is also provided with a pH measuring probe such that the medium acidity can be continuously monitored. Once the pH values in which the lactic acid production decreases are reached, a specific volume of the basic solution should be added to the medium so as to raise the pH values to work levels.

One aspect of the present invention is a system for correcting constant volume acidity in fermentative media consisting of at least one fermenter having at least one sensor for determining real time fermentative medium pH, a filtration module, a vessel for the addition of a base and a base dosimeter, a first pump for pumping fermentative broth from the fermenter to said filtration module, a second pump for pumping the basified fermentative broth fraction back to the fermenter and a heat exchanger, the volumetric ratios of alkaline solution for pH correction to be added to the fraction withdrawn from the fermenter being established by a controller.

Another aspect of the invention is the process for correcting acidity of fermentative media using the inventive system.

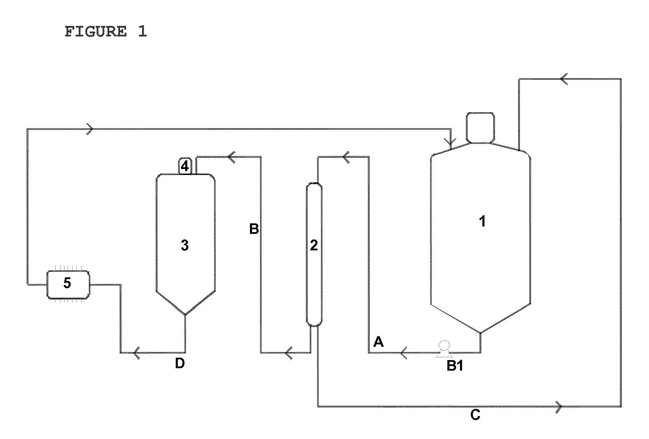

In the present invention, unlike the usual processes of the prior art, the fermenter (1) is connected to a withdrawal, alkalinization and return system of a certain medium volume, as shown in Figure 1. In this scheme, an amount not greater than 12% of the total volume of fermentative medium is removed through actuation of a flow pump (B1), which is responsible for carrying part of the broth (stream (A)) to a filtration module (2) containing a filtering element (not shown).

Stream (A) may have a concentration which is equal or close to that of the fermentation broth at pH values which are equal to or less than the optimal for culture of microorganisms and temperature close to that found inside the fermentation tank. When passing through the filter module (2), a portion of the stream (A) relative to the solids with sizes in the range of 0.5 to 5 $\mu$m, preferably larger than 1 $\mu$m, is retained on said filtering element.

The other portion of the stream is permeated through the filtering element, thus creating stream (B). Stream (B) contains all reaction medium elements present in the fermenter (1) except solids with pH values between 4.0 and 7.0 and temperature between 25°C and 42°C, and non-permeated solids.

The retained solids can be returned to the reaction medium, thus creating stream (C), in order to keep the concentration of microorganisms continuous in the fermentative medium and, in turn, ensuring the highest possible organic acid production.

Stream (C) essentially contains *Lactobacillus sp.* cells, cell debris, and may also contain inorganic solid waste inherent to the fermentative process and to the composition of the medium.

The filtration module (2) should be provided with a filtering agent with pore diameter between 0.5 and 5 $\mu$m, and more preferably 1 $\mu$m. Several types of filtering elements may be used in the filtration system such as bag filters (pouch) preferably comprised of microstructured membranes with tangential filtration system, in order to promote the retention of most solids present in the fermentative medium, and especially the microorganisms, thus enabling the eventual return thereof to the culture medium without causing major losses to the fermentative process.

From the filtering element present in the filtration module (2), stream (B) is conducted to a mixture tank (3) where a certain amount of base will be added through a dosing element (4), in such a way that the pH of the permeated solution (glucose, yeast extract, magnesium sulfate, manganese sulfate, etc.) is adjusted to pH values which are greater than 7, so that when returned to the fermenter (stream (D)) in appropriate amounts, it will be able to produce decreased acidity, i.e. an increased fermentation broth pH within the values required for a good productivity of lactic acid or another organic acid.

Bases which are added to the permeate broth can be both organic and inorganic, preferably KOH, NaOH, $Ca(OH)_2$ or $NH_4OH$. The liquid form base may be manually added or, preferably added through a dosing device (4) positioned on the top of said mixture tank (3).

Before returning to the fermenter, alkaline solution (stream (D)) with the same composition of stream (B) plus base added in the mixture tank (3) with temperatures between 30°C and 60°C and pH values between 6 and 9, preferably greater than 7, can be transported through a heat exchanger (5) which will be responsible for storing the alkaline solution in the process working temperature in order to prevent interference in the fermentative process.

Also, the heat removed from the solution by the cooling fluid of the heat exchanger (5) can be transported into the fermentative tank (1), so as to keep the temperature of said fermentative tank (or of said fermentative tanks) within appropriate values for producing organic acids.

The whole process is digitally controlled in an integrated manner, in order to allow that the fermenter outflow rate, the amount of base added to the solution in (3) by the element (4) and the alkaline solution backflow rate to the reactor (stream (D)) are appropriate and in accordance with the adjusting requirement of the medium pH at a given point in the organic acid production process.

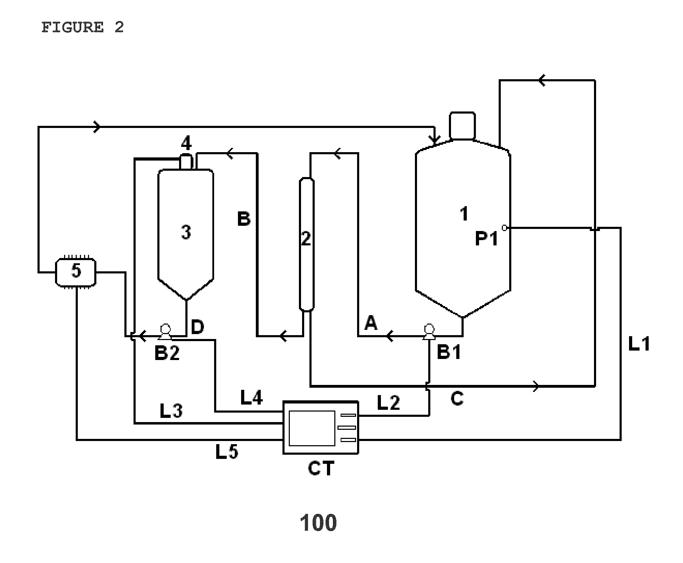

Figure 2 schematically illustrates the control layout used in the described process, so that pumps (B1) and (B2) can provide sufficient flow rate in such a way that the fermentation process occurs within the recommended, or empirically defined, pH value range for the maximum organic acid production.

The system of the invention as illustrated in Figure 2 is generally designated by numeral (100).

The sensing element (P1) is positioned inside the fermentative medium of the fermenter (1) in order to control the variation of pH values in real time during the process, and it may also be an array or set of sensing elements (not shown) which lead to a greater accuracy in measurements. The sensing elements (P1) are commercially available pH sensors. Element (P1) is connected to the controlling element (CT) via the line (L1).

The controlling element (CT) is also responsible for controlling: i) the flow rate of the fermentative medium solution, stream (A), through connection with the pump (B1) via (L2); ii) the flow rate of the basic solution through line (L3); iii) the flow rate of the fermentative medium alkalinized solution, stream (D), through connection with the pump (B2) via (L4) and iv) the flow rate of the cooling liquid in the heat exchanger (5) via (L5) in order to keep the temperature within the process working range.

The controlling element (TC) is comprised of a commercially available device that is well known in the field of industrial process control, which is equipped with a dedicated software (called supervisory system) in order to provide an easy-to-operate interface, as well as a real time control of the process as a whole. Briefly, the CT receives and reads all information from the sensors installed throughout the process, and through programmed instructions in their memory performs specific actions (control of pump flow rates, temperature control, pH control, valve opening and closure) according to real time sensor conditions, thus allowing a high level of automation in the control of several steps of the process under reference.

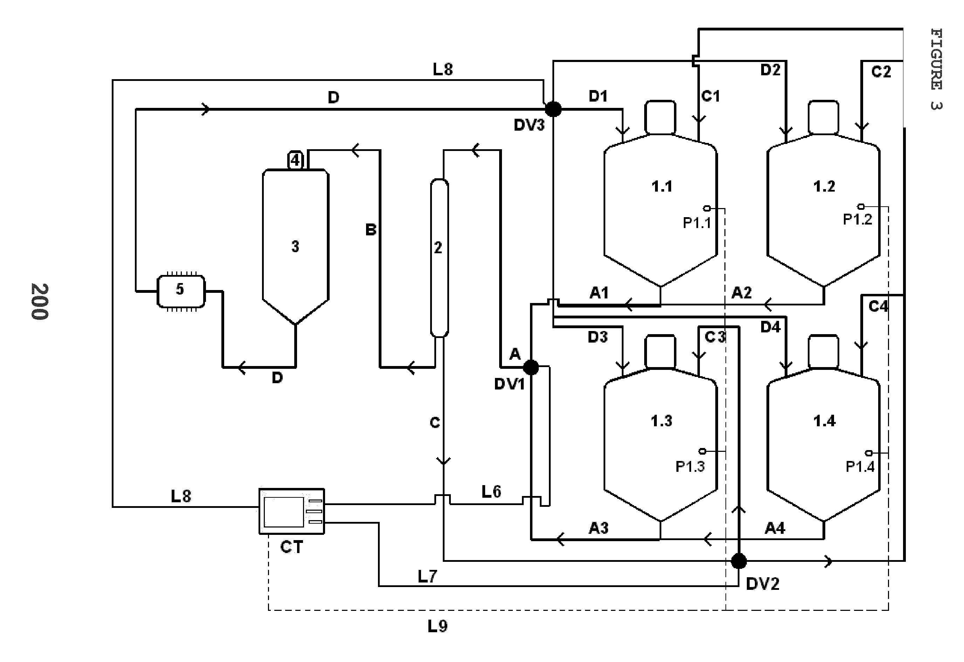

Figure 3 depicts the above-described process which is adapted for a set of reactors, so that the permeate alkalinization process represented by stream (B) can continuously operate while different batches at different fermentative tanks ((1.1), (1.2), (1.3), (1.4) tanks) are carried out alternately.

The system of Figure 3 is generally designated by numeral (200).

Figure 3 depicts an arrangement of two sets of two fermenters in series. However, it should be clear to those skilled in the art that other modifications and variations of these arrangements are possible, without departing from the scope of the invention.

In this Figure, streams (A1), (A2), (A3) and (A4) are received by a suitable control valve, called flow divider (DV1) herein. Said flow divider device is connected to the central control element (TC) through a line (L6) in order to control the outflow of the fermentative media contained in said fermenters (1.1) (1.2) (1.3) and (1.4).

Streams (C1), (C2), (C3) and (C4) are formed by the flow divider device (DV2) from the stream (C) that can be essentially formed by *Lactobacillus sp.* cells or cell debris, and may also contain inorganic solid waste inherent to the fermentative process and the composition of such media. Said flow divider device is also connected to the central control element (CT) through a line (L7) in order to control the inflow of retained solids *(Lactobacillus sp.* cells or cell debris, inorganic solid waste) into said fermenters (1.1), (1.2), (1.3) and (1.4).

Stream (D), consisting of fermentation broth basified in tank (3), is subdivided into (D1), (D2), (D3) and (D4) by a flow divider (DV3), thereby providing the suitable amount of basified broth for each fermenter (1.1), (1.2), (1.3) and (1.4) in a certain process time. Said flow divider device (DV3) is also connected to the central control element (CT) through a line (L8) in order to control the inflow of the basified broth into said fermenters (1.1), (1.2), (1.3) and (1.4).

Streams from said tanks, designated herein as (A1), (A2), (A3) and (A4) streams, are essentially comprised of the fermentation broth contained in respective tanks with temperature and pH values close to the optimal values of organic acid production.

As shown in Figure 2, all fermentation tanks (1.1), (1.2), (1.3) and (1.4) are equipped with sensing elements (P1.1), (P1.2), (P1.3) and (P1.4), and all of them are connected to the control element (CT) through the line (L9), in order to control the pH of each reaction medium in real time.

In operation, while, for example, stream (A1) leaving the fermenter (1.1) is directed via (DV1) to the filtration module (2) to be subjected to separation and thus generating stream (C) of retained solids to be returned to the fermenter (1.1) and stream (B) containing organic acid which will have the pH corrected by the basic solution in vessel (3), fermenters (1.2), (1.3) and (1.4) can be inactive or at a later stage of the process which does not require correction by adding alkaline solution.

The (DV1) function is restricted to the control of the outflow rates of the reaction media, in operation at a certain time in the process.

After filtration, separation and basification, resulting streams (C1) and (D1) are directed to the fermenter (1.1).

Immediately after stream (A1) is separated in the filtration module (2), stream (A2) can be directed to said module (2) without requiring that the entire process is completed before starting a new batch. Thus, the same can be done in continuous mode.

Similarly to the stream (A1), streams (C2), (C3) and (C4) and (D2), (D3) and (D4) which result from the filtration, separation and alkalinization of streams (A2), (A3) and (A4) are respectively directed to the fermenters (1.2), (1.3) and (1.4).

Thus, the described system can continuously operate, 24 hours a day, by alternating the presented fermenters. It should be clear to those skilled in the art that more fermenters can be added to the process without departing from the scope of the invention, such procedure being within the reach of a person skilled in the art.

The present invention will be illustrated by the following Examples, which should not be construed as limiting thereof.

EXAMPLE 1

This is a Comparative Example.

A typical MRS-type fermentative broth comprising:

- 1.0% of peptone;
- 0.8% of animal tissue extract (usually bovine meat);
- 0.4% of yeast extract;
- 2.0% of glucose;
- 0.5% of sodium acetate;
- 0.1% of Polysorbate 80 (also known as Tween 80);
- 0.2% of potassium hydrogen sulfate;
- 0.2% of ammonium citrate;
- 0.02% of magnesium sulfate; and
- 0.005% of manganese sulfate;

as above-described in the present invention, was incubated with *Lactobacillus sp.* cells in a total volume of 1000 L under stirring for 18 hours at 39 °C and initial pH of 7.2. For a productivity in the range of 3.0 kg/h, it was necessary to add 120 L of 7.5 N NaOH aqueous solution so that the pH of the medium was kept at 7.2. Therefore, the increased volume caused a 12% increase in the total volume of the culture medium. Thus, it was necessary to remove more 120 L than necessary from the culture medium in a later step for separating the formed product, namely: organic acids and salts thereof. Consequently, such increased volume generated a 12% increase in the energy consumption of the process which, for the above conditions, amounts to 75.4 KW/h in a daily production plant of 72 kg of organic acid. Thus, it is clear from this Example that the total energetic consumption of the fermentative process is approximately 1.05 kW/h per kg of produced acid.

EXAMPLE 2

This Example illustrates the process of the invention.

A MRS-type fermentative broth, as shown in Example 1 of the present document, made as described in Example 1 and incubated with the same cell type in a 1000 L volume under stirring for 18 hours at 39°C and initial pH of 7.2 in order to keep the production in the range of 3.0 kg/h, has required an amount equivalent to 120 L of the reaction volume to be removed, filtered and alkalinized with 36 Kg of NaOH and then fed back to the reaction medium, and occurring with an energy consumption equivalent to 0.67 KW/h including all process transport (pumps), filtration, dosing, monitoring and control steps. According to the present Example, it can be observed that the total amount of saved energy in a plant for the production of 72 kg of organic acid per day will be approximately of 74.7 KW/h, resulting in a power consumption of 1.03 kW/h per kg of produced acid. That is, 11.8% of savings in energy consumption in a subsequent process of separation and purification of the production medium.

EXAMPLE 3

This Example illustrates the substantial energy savings provided by the process of the invention when applied to a system of several reactors.

The withdrawal, filtration and alkalinization system of the fermentation broth was connected to a set of 4 (four) fermentative reactors (1.1, 1.2, 1.3 and 1.4), as shown in Figure 3 of the present specification. The reactors were put in operation with a difference of 6 hours between the beginning of each process, so that the broth withdrawn from a fermenter could be used in another fermenter, which leads to a proportional decrease in the circulating volume in the alkalinization process equivalent to 7.5% of the total volume incubated in the four reactors, versus the 12% used in the single reactor system. Considering a total production of 12.0 kg/h, the total energy consumption of the process was equivalent to 1.65 KW/h, including all process transport (pumps), filtration, dosing, monitoring and control steps. According to the present Example, it can be observed that the total amount of saved energy in a subsequent step of purification due to decreased volume in a process for the production of 288 kg of organic acid per day is 113 KW/h, leading to an energy consumption rate of 0.4 KW/h per kg of produced acid. That is, 37.5% of savings in energy consumption of the process as a whole.

**Claims**

1.  System for correcting constant volume acidity of fermentative media for the production of organic acids **characterized by** comprising:

    a) at least one fermenter (1) having a sensor (P1) for determining real time fermentative medium pH;
    b) a filtration module (2);
    c) a first pump (B1) for pumping fermentative broth, stream (A), from said fermenter (1) to said filtration module (2);
    d) a vessel (3) for the addition of a base having a base dosing element (4);
    e) a second pump (B2) for pumping the basified fermentative broth fraction, stream (D), back to the fermenter (1); and
    f) a heat exchanger (5),

    wherein the volumetric ratios of alkaline solution for pH correction to be added to the fraction withdrawn from the fermenter are established by a controller (CT) connected to said at least one sensor (P1), said first pump (B1), said base dosing element (4), said second pump (B2) and said heat exchanger (5) via lines (L1), (L2), (L3), (L4) and (L5), respectively.

2.  System, according to claim 1, **characterized in that** the filtration module (2) is provided with a filtering element.

3.  System, according to claim 2, **characterized in that** the pore diameter of said filtering element is between 0.5 $\mu$m and 5 $\mu$m.

4.  System, according to claim 3, **characterized in that** the pore diameter of said filtering element is preferably of 1 $\mu$m.

5.  System, according to any one of claims 2 to 4, **characterized in that** the filtering element is of the bag filter type comprising microstructured membranes with tangential filtration system.

6.  System, according to claim 1, **characterized in that** an amount up to 12% of the fermentative medium volume contained in the fermenter (1) is removed with the aid of the pump (B1) to be alkalinized.

7.  System, according to claim 1, **characterized by** being continuously operated.

8.  System, according to claim 1, **characterized by** being used in the production of organic acids.

9.  System, according to claim 8, **characterized in that** the organic acid is lactic acid.

10. System, according to claim 1, **characterized in that** a single filtration, alkalinization and temperature adjustment system is used in a set of at least two fermenters continuously or batchwise operated.

11. System, according to claim 10, **characterized by** comprising at least two sets of fermenters (1.1, 1.2, 1.3 and 1.4) continuously operating in series, while different batches in different fermenters are carried out alternately.

12. System, according to claim 11, **characterized by** receiving streams A1, A2, A3 and A4 through a flow divider (DV1) connected to the central control element (CT) via a line (L6), in order to control the outflow rates of the fermentative media contained in fermenters (1.1), (1.2), (1.3) and (1.4).

13. System, according to claim 11, **characterized by** subdividing stream (C) through a flow divider (DV2) connected to the central control element (CT) via a line (L7), in order to control the outflow rates of solid waste in fermenters (1.1), (1.2), (1.3) and (1.4).

14. System, according to claim 11, **characterized by** subdividing stream (D) through a flow divider (DV3) connected to the central control element (CT) via a line (L8), so as to control the outflow rates of basified broth in fermenters (1.1), (1.2), (1.3) and (1.4) in order to provide the appropriate amount of said broth to each fermenter (1.1), (1.2), (1.3) and (1.4) in a certain process time.

15. System, according to claim 11, **characterized in that** the fermenters (1.1), (1.2), (1.3) and (1.4) are equipped with sensing elements (P1.1), (PI.2) (P1.3) and (P1.4) connected to said control element (CT) via a line (L9), in order to control the reaction medium pH of each of said fermenters in real time.

16. System, according to claim 11, **characterized by** being operated in such a way that while stream (A1) leaving the fermenter (1.1) is directed via (DV1) to the filtration module (2) to be subjected to separation and thus generating stream (C) of the fermentative medium to be returned to the fermenter (1.1) and stream (B) containing organic acid which will have the pH corrected by the basic solution in vessel (3), fermenters (1.2), (1.3) and (1.4) are inactive or at another stage of the process which does not require correction by adding alkaline solution.

17. Process for correcting constant volume acidity in fermentative media to be effected with the aid of the system as defined in any one of claims 1 to 16, **characterized by** comprising the steps of:

   a) determining the fermentation medium pH with the aid of a sensor (P1) installed on the at least one fermenter (1);
   b) directing, with the aid of a pump (B1) via stream (A), up to 12% of the volume of said fermenter (1) towards a filtration module (2) in order to filter said stream (A), thus obtaining a filtrate stream (B) and a stream (C) of retained solids;
   c) recirculating the solids retained in the filtering element via stream (C) to said at least one fermenter (1), so as to keep the microorganism concentration constant in the fermentative medium while the filtrate stream (B) is directed into a vessel (3) having a base dosing element (4) to adjust the pH of the fermentative medium fraction withdrawn from said fermenter (1), thus obtaining a fermentative medium stream (D) with adjusted pH; and
   d) directing, with the aid of a pump (B2), said fermentative medium stream (D) with adjusted pH to a heat exchanger (5) and from this to the at least one fermenter (1), restarting the cycle,

   wherein the controller (CT) allows that the fermenter (1) outflow rate, the amount of base added to the solution in said addition vessel (3) and the alkaline solution backflow rate, stream (D), to said fermenter (1) are appropriate and in accordance with the adjusting requirement of the medium pH at a given point in the organic acid production process.

18. Process, according to claim 17, **characterized by** using KOH, NaOH, Ca(OH)$_2$, NH$_4$OH in the vessel (3) to adjust the pH of the fermentative broth.

19. Process, according to claim 18, **characterized by** using sodium hydroxide in the vessel (3) for base addition.

20. Process, according to claim 17, **characterized by** being used in the production of organic acids.

21. Process, according to claim 20, **characterized in that** the organic acid is lactic acid.

FIGURE 1

FIGURE 2

100

FIGURE 3

200

## INTERNATIONAL SEARCH REPORT

| International application No. |
| --- |
| **PCT/BR2011/000287** |

| A. | CLASSIFICATION OF SUBJECT MATTER |
| --- | --- |

**C12M1/00 (2006.01), C12P7/40 (2006.01)**

According to International Patent Classification (IPC) or to both national classification and IPC

| B. | FIELDS SEARCHED |
| --- | --- |

Minimum documentation searched (classification system followed by classification symbols)

**C12M1/00, C12P7/40**

Documentation searched other than minimum documentation to the extent that such documents are included in the fields searched

Electronic data base consulted during the international search (name of data base and, where practicable, search terms used)

**Epodoc, Espacenet**

| C. | DOCUMENTS CONSIDERED TO BE RELEVANT | |
| --- | --- | --- |
| Category* | Citation of document, with indication, where appropriate, of the relevant passages | Relevant to claim No. |
| Y | EP 1496108 A1 ( BARLEY FERMENTATION TECHNOLOGI [JP]) 12 January 2005 (12.01.2005) (paragraphs 5-8, 12, 15, 16, 17, 29, 31 and figure 3) | 1 to 21 |
| Y | WO 2009006909 A1 ( KOLBAROV VAMBOLA VIJACHESLAVOV [EE]) 15 January 2009 (15.01.2009) (page 1, lines 3-5; page 2, lines 10-21; page 3, lines 18-20; example in page 4, line 21 and page 5, line 2; figure 1) | 1 to 21 |
| Y | US 6596521 B1 (KOREA ADVANCED INST SCI TECH [KR]) 22 July 2003 (22.07.2003) (column 4, lines 7-55; column 5, lines 46-57; Example 2 and 3) | 11 to 21 |

| ☒ | Further documents are listed in the continuation of Box C. | ☒ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **16/12/2011** | **231211** |

| Name and mailing address of the ISA/ INSTITUTO NACIONAL DA PROPRIEDADE INDUSTRIAL Rua Mayrink Veiga nº 9, 18º andar cep: 20090-050, Centro - Rio de Janeiro/RJ Facsimile No. +55 21 3037-3663 | Authorized officer **Ellelton Rezende Coelho** Telephone No. +55 21 3037-3493/3742 |
| --- | --- |

Form PCT/ISA/210 (second sheet) (July 2009)

**INTERNATIONAL SEARCH REPORT**

| | International application No. |
|---|---|
| | **PCT/BR2011/000287** |

C (Continuation).    DOCUMENTS CONSIDERED TO BE RELEVANT

| Category* | Citation of document, with indication, where appropriate of the relevant passages | Relevant to claim No. |
|---|---|---|
| A | FR 2686897 A1 (SONERTEC [FR])<br>06 August 1993 (06.08.1993)<br>(the whole document)<br>-------------------------------------------- | --- |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5766439 A
- US 20040033573 A
- US 4882277 A
- US 20060094093 A
- US 5510526 A
- WO 9919290 A

### Non-patent literature cited in the description

- **BENNINGA.** H. history of making lactic acid: a chapter in the history of biotechnology. Kluwer Academic Press, 1989
- *World Journal of Microbiology & Biotechnology,* 1997, vol. 13